# EUROPEAN PATENT APPLICATION

(11) **EP 4 814 526 A1**
(43) Date of publication of application: **30.09.2026**
(21) Application number: 26168388.2
(22) Date of filing: 27.03.2026
(51) Int. Cl.: G01R 33/56, G06N 3/0895, G06V 10/82, G16H 30/00

(54) **SELF-SUPERVISED CONTRASTIVE LEARNING FRAMEWORK FOR MEDICAL IMAGE CLASSIFICATION**

(30) Priority: 28.03.2025 US 202563779450 P; 19.05.2025 US 202519211482
(71) Applicant: Siemens Healthineers AG, 91301 Forchheim (DE)
(72) Inventor: IYER, Kritika, Malvern, PA 19355-1418 (US); SHINAGAWA, Yoshihisa, Malvern, PA 19355-1418 (US); FARHAND, Sepehr, Malvern, PA 19355-1418 (US); HERMOSILLO VALADEZ, Gerardo, Malvern, PA 19355-1418 (US); WANG, Yuli, Jersey City, NJ 07302 (US)
(74) Representative: HKW Intellectual Property PartG mbB

(57) **Abstract**

An image processing system is provided. The image processing system comprises one or more non-transitory computer-readable media for storing computer-readable program code. The image processing system comprises a processor device in communication with the one or more non-transitory computer-readable media. The processor device being operative with the computer-readable program code to perform steps including receiving unlabeled medical image data, identifying one or more image acquisition parameters by applying the unlabeled medical image data to at least one pre-trained contrastive network, and providing the one or more image acquisition parameters.

## Description

The present framework relates to medical image classification using self-supervised contrastive learning.

Magnetic Resonance (MR) imaging provides clinical experts
with detailed three-dimensional (3D) visualization and analysis of a patient's organ tissues, enabling the detection of abnormalities. MR images are generated by recording the signal intensities emitted by water protons in tissue when excited by a resonant electromagnetic radio-frequency field. This process produces images with visible contrast between tissue elements (e.g., fat, fluids) by exploiting their varying characteristics, such as proton density and relaxation times. Typically, a comprehensive understanding of abnormalities is achieved by analyzing multiple MR image sequences, which may be referred to according to the dominant influence on the appearance of tissues. Exemplary MR image sequences include T1-weighted, T2-weighted, Fluid Attenuated Inversion Recovery (FLAIR), Diffusion-Weighted Imaging (DWI), derived Apparent Diffusion Coefficient (ADC) maps, etc.

During the acquisition of these MR image sequences, relevant patient and imaging information is stored in the Digital Imaging and Communications in Medicine (DICOM) header, including fields such as "Body Part Examined," "Procedure Step Description," "Series Description," and "Protocol Name." However, these fields often lack informative description specific to sequence types, and discrepancies may arise. Such discrepancies occur frequently, especially when default scanner protocol information is used, and technologists do not have sufficient time to update every DICOM field accurately during a busy clinical day. These fields are often incomplete, error-prone, and inconsistent across hospital sites and scanner vendors.

This issue is further compounded by the diversity of MR imaging protocols and parametric sequences across different institutions globally. Additionally, the use of multiple MR imaging scanners from various manufacturers introduces extensive variations in voxel intensity distributions across sequences. Consequently, radiologists and referring physicians must navigate these inconsistencies while performing thorough evaluations of disease status. Such variations can also disrupt radiologists' hanging protocols for reading images in a Picture Archiving and Communication System (PACS), often necessitating manual intervention to correct discrepancies. These inconsistencies present significant challenges when constructing large-scale clinical cohorts for patients who have undergone specific MR imaging types or when developing artificial intelligence (AI) algorithms tailored for medical imaging.

An automated method for classifying MR image sequences can significantly reduce the need for radiologists to manually oversee them, enhancing efficiency and accuracy. However, previous approaches rely heavily on DICOM header data for accurate categorization and supervision. Moreover, achieving high classification accuracy and model generalizability demands a large quantity of labeled data, which may not always be readily available and requires time-consuming manual verification to ensure label accuracy.

The present framework relates to medical image classification using self-supervised contrastive learning. The framework identifies one or more image acquisition parameters by applying unlabeled medical image data to at least one pre-trained contrastive network. The one or more identified image acquisition parameters are then provided.

According to one aspect, an image processing system is provided. The image processing system comprises one or more non-transitory computer-readable media for storing computer-readable program code. The image processing system comprises a processor device in communication with the one or more non-transitory computer-readable media. The processor device being operative with the computer-readable program code to perform steps including
a) receiving unlabeled medical image data,
b) identifying one or more image acquisition parameters by applying the unlabeled medical image data to at least one pre-trained contrastive network, and
c) providing the one or more image acquisition parameters.

The one or more image acquisition parameters may comprise a type of an imaging modality used in acquiring the unlabeled medical image data, one or more parameter settings of the imaging modality, or a combination thereof.

The one or more image acquisition parameters may comprise repetition time (TR), echo time (TE), inversion time, flip angle, acceleration, or a combination thereof.

The processor device may be operative with the computer-readable program code to identify the one or more image acquisition parameters by identifying a magnetic resonance (MR) sequence used in acquiring the unlabeled medical image data.

The magnetic resonance sequence may comprise T1 weighted, T2 weighted, Fluid-Attenuated Inversion Recovery (FLAIR), Time-of-Flight (TOF), Trace-Weighted Imaging (TraceW), Diffusion-Weighted Imaging (DWI), ADC, Gradient Echo Imaging (GRE), Perfusion MR image sequence, or a combination thereof.

The at least one pre-trained contrastive network may comprise a simple framework for contrastive learning of visual representations (SimCLR). The at least one pre-trained contrastive network may comprise a Simple Siamese Network (SimSiam).

The processor device may be further operative with the computer-readable program code to train the contrastive network using self-supervised learning based on a training dataset of unlabeled images. The training dataset of unlabeled images may be augmented by performing flip, rotation and elastic deformation. The training dataset of unlabeled images may be downsized by resampling at a lower resolution.

The processor device may be further operative with the computer-readable program code to fine-tune the contrastive network in a supervised manner using labeled training image data.

According to another aspect a computer-implemented method for image processing is provided. The computer-implemented method for image processing comprises
a) receiving unlabeled medical image data;
b) identifying one or more image acquisition parameters by applying the unlabeled medical image data to at least one pre-trained contrastive network; and
c) providing the one or more image acquisition parameters.

Identifying the one or more image acquisition parameters by applying the unlabeled medical image data to the at least one pre-trained contrastive network may comprise classifying the unlabeled medical image data according to a type of magnetic resonance (MR) sequence used to acquire the unlabeled medical image data. Identifying the one or more image acquisition parameters by applying the unlabeled medical image data to the at least one pre-trained contrastive network may comprise applying the unlabeled medical image data to a simple framework for contrastive learning of visual representations (SimCLR). Identifying the one or more image acquisition parameters by applying the unlabeled medical image data to the at least one pre-trained contrastive network may comprise applying the unlabeled medical image data to Simple Siamese Network (SimSiam).

According to another aspect, the method further comprises displaying a representation of the medical image data. Displaying the representation may comprise
a) receiving the medical image data, in particular from a database,
b) determining one or more image acquisition parameters, in particular according any one of the preceding aspects,
c) generating a representation of the medical image data for displaying in a user interface based on the one or more image acquisition parameters,
d) displaying the representation in the user interface.

The representation may be generated by processing the medical image data wherein the processing depends on the image acquisition parameters.

The representation may comprise one or more two-dimensional representation images rendered from the medical image data. The representation images may comprise a plurality of image pixels. In particular, the representation images may be two-dimensional renderings of the medical image or of different views of the medical image. Two-dimensional renderings may, in general, rely on known rendering procedures, including ray-casting, ray-tracing, texture-rendering or the like. Thereby, the views and the rendering may depend on the image acquisition parameters. For instance, the image acquisition parameters may suggest a certain rendering or pre-processing and/or a particular view.

Further, the representation may comprise a graphical user interface in which the representation images are included at a predefined position. In particular, the graphical user interface may be configured to derive a medical diagnosis based on the medical image. Via the graphical user interface, the user may inspect the medical image, make measurements and record a medical diagnosis (e.g., in the form of a medical report).

By automatically generating the representation based on the image acquisition parameters views are automatically generated and offered to a user which will likely be required based on the image acquisition parameters.

According to an aspect, the step of generating comprises determining a displaying setting based on the image acquisition parameters and applying the selected displaying setting for generating the representation, the displaying setting being selected from: a contrast setting, a brightness, an intensity windowing, an image enhancement, a look-up table, a viewing plane, a segmentation mask, a zoom level or panning, and/or a volumetric rendering parameter.

According to an aspect, the method further comprises selecting, based on the image acquisition parameters, a hanging protocol including a rule set for displaying one or more representations of a medical image data in a user interface, wherein, in the step of displaying, the representation is displayed based on the hanging protocol.

Current reading and reporting systems use general techniques known as "hanging protocols" to format the display or layout of medical images or excerpts from medical images. Hanging protocols allow a user to specifically set displaying environments according to modality, anatomy, and procedure. Hanging protocols present one or more perspectives or views (e.g., in the form of the aforementioned representations) of the medical image to a user, such as a radiologist. Representations may be grouped and located in a graphical user according to characteristics such as the image acquisition parameters. In addition, hanging protocols may comprise rules or instructions for obtaining additional information such as comparative medical images acquired before or after the medical image or for applying certain image analysis tools.

According to some examples, the hanging protocol may be selected from a plurality of predefined hanging protocols. The predefined hanging protocols may be configured according to different use case and the step of selecting may comprise identifying a use case based on the image acquisition parameters and selecting the hanging protocol corresponding to the identified use case.

By selecting the appropriate hanging protocol, the user is automatically provided with a user interface specifically adapted for the image data and the diagnostic task.

The computer-implemented method may comprise training the contrastive network using self-supervised learning based on a training dataset of unlabeled images. The computer-implemented method may further comprise fine-tuning the contrastive network in a supervised manner using labeled training image data.

Providing the one or more image acquisition parameters may comprise storing the one or more image acquisition parameters in the medical image data for subsequent retrieval. Providing the one or more image acquisition parameters may comprise processing or presenting the medical image data based on the one or more image acquisition parameters.

According to an aspect, the method may comprise retrieving, from a database and based on the one or more image acquisition parameters, a comparative medical image, processing the comparative medical image so as to generate a comparative representation for displaying in the user interface, and displaying the comparative representation in the user interface.

According to some examples, the comparative medical image may have the same or similar image acquisition parameters as the medical image. According to some examples, the comparative medical image may be processed in the same way as the medical image data. For instance, the comparative medical images may be based on the same magnetic resonance sequence and/or image weighting as the medical image data. With that, the medical image data can be more readily compared to the medical image data by the user and/or the same measurements may be automatically made.

The comparative medical image may relate to the same patient as the medical image data. The comparative medical image may relate to a different patient as the medical image data. In particular, the comparative medical image may have a degree of similarity to the medical image data. The comparative medical image may be obtained from a database of comparative medical images. In particular, the comparative medical image may be obtained from an electronic medical textbook. The comparative medical image may be associated with a verified medical diagnosis.

By offering a comparative medical image to a user, the user can be provided with additional information for deriving a medical diagnosis.

According to some examples, the medical image data was acquired of the patient at a first point in time and the comparative medical image was acquired of the patient at a second point in time different than the first point in time.

In other words, a prior or subsequent medical image of the patient may be automatically retrieved which has comparable image acquisition parameters. In particular, this may mean that the medical image data shows a body part of the patient at the first point in time and the comparative medical image shows the body part at a second point in time. With that, the user can more easily determine a development of the health condition of the patient and can come up with a better diagnosis. In this regard, since the comparative medical image is automatically retrieved based on the one or more image acquisition parameters, the user is spared from the time-consuming task of having to search the database for appropriate priors.

According to some examples, the method further comprises selecting, based on the one or more image acquisition parameters, a reporting template for producing a medical report corresponding to the medica image, and providing the reporting template via the user interface.

A reporting template may be a pre-configured data structure or building block or module on the basis of which a structured medical report may be generated. A medical report may be generated based on at least one reporting template.

Selecting the reporting template may comprise a selection from a plurality of reporting templates. Each reporting may be specific to a certain image acquisition information. For instance, a certain reporting template may be associated to magnetic resonance scan of the brain, while another reporting template is associated to a chest CT scan.

Each reporting template may specify one or more data fields which have to be addressed or filled for completing the medical report. Further, a reporting template may comprise one or more pull-down menus with items a user can select. As such, a reporting template may also be conceived as an input form or mask structuring the information to be provided for a given diagnostic task.

According to some examples, the method may further comprise pre-filling the reporting template based on the one or more image acquisition parameters and/or any other image processing results, e.g., as obtained by applying image processing tools.

By fetching appropriate reporting templates, the user is automatically provided with appropriate template data structures. In turn, the user is relieved from the burden of having to search for correct template data structure on her or his own in potentially vast databases.

According to one aspect, the one or more non-transitory computer-readable media comprising machine readable instructions, that when executed by one or more processor devices, cause the one or more processor devices to perform method steps comprising:
a) receiving unlabeled medical image data;
b) identifying one or more image acquisition parameters by applying the unlabeled medical image data to at least one pre-trained contrastive network; and
c) providing the one or more image acquisition parameters.

A more complete appreciation of the present disclosure and many of the attendant aspects thereof will be readily obtained as the same becomes better understood by reference to the following detailed description when considered in connection with the accompanying drawings.
FIG. 1 is a block diagram illustrating an exemplary computer system;
FIG. 2 shows an exemplary image processing method;
FIG. 3 shows exemplary unlabeled input MR images;
FIG. 4 shows an exemplary simple framework for contrastive learning of visual representations (SimCLR) architecture and an exemplary Simple Siamese Networks (SimSiam) architecture;
FIG. 5a shows an exemplary visualization of the latent space of an exemplary SimCLR model at epoch 50 of self-supervised pre-training;
FIG. 5b shows an exemplary visualization of the latent space of the exemplary SimCLR model after supervised fine-tuning;
FIG. 6a shows exemplary datasets used for model fine-tuning and evaluation;
FIG. 6b shows exemplary classification accuracy results for new MR image sequences from the extracted datasets;
FIG. 6c shows various exemplary classification graphs illustrating the MR sequence classification accuracy performance of an exemplary SimSiam model;
FIG. 7a shows an exemplary table depicting the MR sequence classification performance of an exemplary SimSiam model across varying fine-tuning percentages and batch sizes; and
FIG. 7b shows an exemplary table depicting the MR sequence classification performance of an exemplary SimCLR model across varying fine-tuning percentages and batch sizes

### DETAILED DESCRIPTION

In the following description, numerous specific details are set forth such as examples of specific components, devices, methods, etc., in order to provide a thorough understanding of implementations of the present framework. It will be apparent, however, to one skilled in the art that these specific details need not be employed to practice implementations of the present framework. In other instances, well-known materials or methods have not been described in detail in order to avoid unnecessarily obscuring implementations of the present framework. While the present framework is susceptible to various modifications and alternative forms, specific embodiments thereof are shown by way of example in the drawings and will herein be described in detail. It should be understood, however, that there is no intent to limit the invention to the particular forms disclosed, but on the contrary, the intention is to cover all modifications, equivalents, and alternatives falling within the spirit and scope of the invention. Furthermore, for ease of understanding, certain method steps are delineated as separate steps; however, these separately delineated steps should not be construed as necessarily order dependent in their performance. Independent of the grammatical term usage, individuals with male, female or other gender identities are included within the term."

Unless stated otherwise as apparent from the following discussion, it will be appreciated that terms such as "identifying", "providing", "segmenting," "generating," "registering," "determining," "aligning," "positioning," "processing," "computing," "selecting," "estimating," "detecting," "tracking" or the like may refer to the actions and processes of a computer system, or similar electronic computing device, that manipulates and transforms data represented as physical (e.g., electronic) quantities within the computer system's registers and memories into other data similarly represented as physical quantities within the computer system memories or registers or other such information storage, transmission or display devices. Embodiments of the methods described herein may be implemented using computer software. If written in a programming language conforming to a recognized standard, sequences of instructions designed to implement the methods can be compiled for execution on a variety of hardware platforms and for interface to a variety of operating systems. In addition, implementations of the present framework are not described with reference to any particular programming language. It will be appreciated that a variety of programming languages may be used.

The automatic identification of Magnetic Resonance Imaging (MRI) sequences can streamline clinical workflows by reducing the time radiologists spend manually sorting and identifying sequences, thereby enabling faster diagnosis and treatment planning for patients. However, the lack of standardization in the parameters of MR image scans poses challenges for automated systems and complicates the generation and utilization of datasets for machine learning research.

One aspect of the present framework provides automatic multi-parametric MR sequence identification using a self-supervised (or unsupervised) contrastive deep learning framework. In some implementations, a contrastive neural network is trained through self-supervised learning to classify multiple common MR image sequence types (e.g., T1w, T2w, Fluid-Attenuated Inversion Recovery (FLAIR), Time-of-Flight (TOF), Trace-Weighted Imaging (TraceW), Diffusion-Weighted Imaging (DWI), ADC, Gradient Echo Imaging (GRE), Perfusion). The neural network requires only two-dimensional (2D) slices for training. The neural network may be applied to different anatomical regions, such as the brain, chest, and abdomen. Advantageously, efficient and accurate classification may be achieved, enabling a streamlined radiology workflow.

In some implementations, the contrastive model is subsequently fine-tuned in a supervised manner to regress one or more image acquisition parameters. The benefit of the contrastive model is that it can learn distinguishing features of different image types using a large unlabeled dataset, which enables better performance when fine-tuning on various downstream tasks with small, labeled datasets. These and other features and advantages will be described in more detail herein.

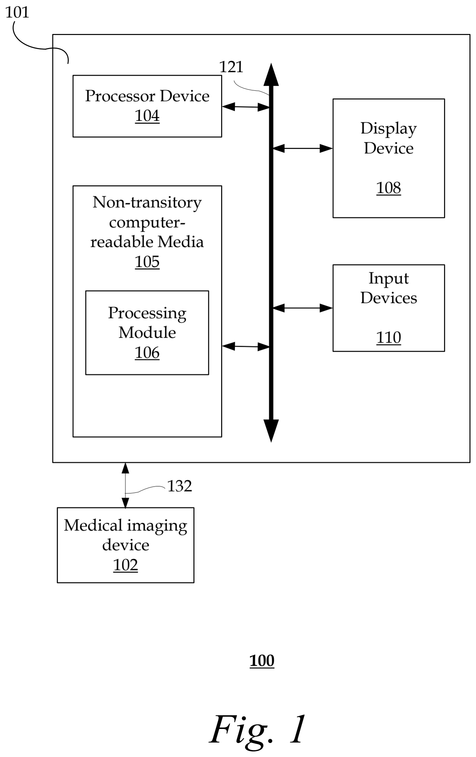

FIG. 1 is a block diagram illustrating an exemplary computer system 101 for implementing the image processing framework as described herein. In some implementations, computer system 101 operates as a standalone device. In other implementations, computer system 101 is connected to other machines, such as medical imaging device 102. In a networked deployment, computer system 101 may operate as a peer machine in a peer-to-peer (or distributed) network environment. Any number of computer systems 101 may be provided (e.g., one, two, three or more) to serve one or more medical imaging devices 102.

Computer system 101 may include a processor device or central processing unit (CPU) 104 coupled to one or more non-transitory computer-readable media 105 (e.g., computer storage or memory device), display device 108 and input devices 110 (e.g., mouse, touchpad or keyboard) via an input-output interface 121. Computer system 101 may further include support circuits such as a cache, a power supply or battery, clock circuits and a communications bus (not shown). Various other peripheral devices, such as additional data storage devices and printing devices, may also be connected to the computer system 101.

The present technology may be implemented in various forms of hardware, software, firmware, special purpose processors, or a combination thereof, either as part of the microinstruction code or as part of an application program or software product, or a combination thereof, which is executed via the operating system. In some implementations, the techniques described herein are implemented as computer-readable program code tangibly embodied in one or more non-transitory computer-readable media 105. In particular, the present techniques may be implemented by a processing module 106. Non-transitory computer-readable media 105 may include random access memory (RAM), read-only memory (ROM), magnetic floppy disk, flash memory, and other types of memories, or a combination thereof. The computer-readable program code is executed by processor device 104 to process, for example, data 132 acquired by medical imaging device 102. The computer-readable program code is not intended to be limited to any particular programming language and implementation thereof. It will be appreciated that a variety of programming languages and coding thereof may be used to implement the teachings of the disclosure contained herein. The same or different computer-readable media 105 may be used for storing a database.

Medical imaging device 102 acquires medical image data 132. Such medical image data 132 may be processed by processing module 106. Medical imaging device 102 may be a radiology scanner and/or appropriate peripherals (e.g., keyboard, display device) for acquiring, collecting and/or storing such medical image data 132. Medical imaging device 102 may acquire medical image data 132 from a subject or patient by using techniques such as magnetic resonance (MR) imaging. Other types of imaging techniques, such as high-resolution computed tomography (HRCT), computed tomography (CT), helical CT, X-ray, angiography, positron emission tomography (PET), fluoroscopy, ultrasound, single photon emission computed tomography (SPECT), or a combination thereof, may also be useful. Medical imaging device 102 may be controlled using a medical imaging software.

It is to be further understood that, because some of the constituent system components and method steps depicted in the accompanying figures can be implemented in software, the actual connections between the system components (or the process steps) may differ depending upon the manner in which the present framework is programmed. Given the teachings provided herein, one of ordinary skill in the related art will be able to contemplate these and similar implementations or configurations of the present framework.

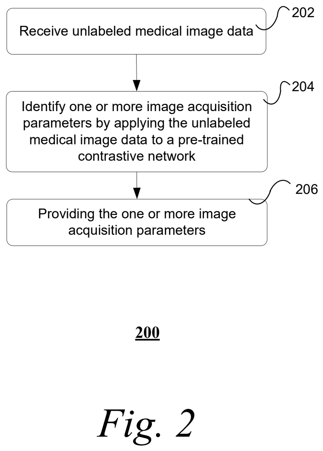

FIG. 2 shows an exemplary image processing method 200. It should be understood that the steps of the method 200 may be performed in the order shown or a different order. Additional, different, or fewer steps may also be provided. Further, the method 200 may be implemented with the system 100 of FIG. 1, a different system, or a combination thereof.

At 202, processing module 106 receives unlabeled medical image data. The medical image data may be unlabeled because one or more image acquisition parameters are not known or correctly (or consistently) identified. For example, there may be a wide range of image acquisition parameters that refer to a particular image sequence type, and this image sequence type may not be correctly and consistently recorded in the DICOM metadata of the medical image data. The unlabeled medical image data may be acquired by and/or received from, for example, medical imaging device 102. The unlabeled medical image data may be three-dimensional (3D) and/or made up of a number of slices, i.e., two-dimensional (2D) medical images. The 2D medical images may be assembled to form a volumetric medical image. An anatomical part of the patient may be imaged. The anatomical part is to be understood as a collection of tissues joined in a structural unit to serve a common function. Examples of anatomical parts include, for example, the brain, chest, abdomen and pelvis.

The image acquisition parameter generally refers to the type of the imaging modality used and/or parameter settings of the imaging modality used to acquire the medical image data. For example, image acquisition parameters may include the following information: type Chest-CT scan, bolus agent: xyz, modality: Siemens Healthineers CT scanner, model number: 12345, kilovoltage peak: xxx, milliampere seconds: yyy. In some implementations, the medical image data has been acquired using a magnetic resonance (MR) medical imaging modality, and the image acquisition parameter relates to the magnetic resonance sequence used in the acquisition procedure. The image acquisition parameters may further include repetition time (TR), echo time (TE), inversion time, flip angle, acceleration and/or other MR imaging parameters.

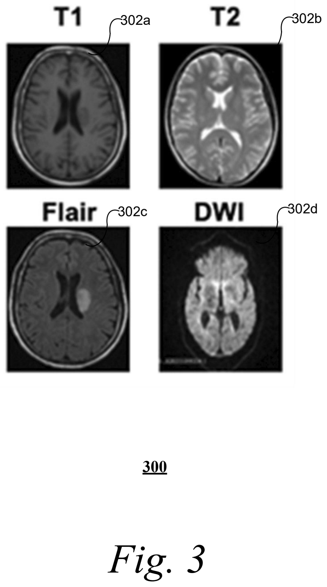

FIG. 3 shows exemplary unlabeled input MR images 302a-d. MR image 302a is acquired using a T1-weighted MR sequence, while MR image 302b is acquired using a T2-weighted MR sequence. MR image 302c is acquired using a Fluid-Attenuated Inversion Recovery (Flair) MR sequence, and MR image 302d is acquired using a Diffusion-Weighted Imaging (DWI) MR sequence.

Returning to FIG. 2, at 204, processing module 106 identifies one or more image acquisition parameters of the unlabeled medical image data by applying the medical image data to a pre-trained contrastive network. In some implementations, identifying the one or more image acquisition parameters includes classifying the medical image data according to the type of magnetic resonance (MR) sequence used to acquire the medical image. The type of MR sequence may include, but are not limited to, T1 weighted, T2 weighted, Fluid-Attenuated Inversion Recovery (FLAIR), Time-of-Flight (TOF), Trace-Weighted Imaging (TraceW), Diffusion-Weighted Imaging (DWI), ADC, Gradient Echo Imaging (GRE), Perfusion MR image sequence, or a combination thereof. Other types of MR image sequences may also be used.

The MR sequence type may be based on image weighting. Image weighting may indicate which relaxation effect the MR imaging was focused on. Each tissue returns to its equilibrium state after excitation by independent relaxation processes of T1 (spin-lattice; that is, magnetization in the same direction as the static magnetic field) and T2 (spin-spin; transverse to the static magnetic field). To create a T1-weighted image, magnetization is allowed to recover before measuring the MR signal by changing the repetition time. This image weighting is useful for assessing the cerebral cortex, identifying fatty tissue, characterizing focal liver lesions, and in general, obtaining morphological information, as well as for post-contrast imaging. To create a T2-weighted image, magnetization is allowed to decay before measuring the MR signal by changing the echo time. This image weighting is useful for detecting edema and inflammation, revealing lesions and abnormalities.

Within the T1 /T2 weightings, there may be more subtle variations. The T2* weighting builds on a distribution of resonance frequencies around the ideal. Over time, this distribution can lead to a dispersion of the distribution of magnetic spin vectors. This results in dephasing. For molecules that are not moving, the deviation from ideal relaxation is consistent over time, and the signal can be recovered by performing a spin echo experiment. T2*-weighted sequences are used to detect deoxygenated hemoglobin, methemoglobin, or hemosiderin in lesions and tissues. Diseases with such patterns include intracranial hemorrhage, arteriovenous malformation, cavernoma, hemorrhage in a tumor, punctate hemorrhages in diffuse axonal injury, superficial siderosis, thrombosed aneurysm, phleboliths in vascular lesions, and some forms of calcification.

The magnetic resonance sequence may relate to the succession of pulse sequences and pulsed field gradients a specimen is subjected to. By varying the parameters of the pulse sequence, different contrasts may be generated between tissues based on the relaxation properties. In other words, different image weightings may be generated. Moreover, different weightings are possible for different sequences. For instance, BLADE may be combined with a T1, T2, or STIR weighting.

In some implementations, the unlabeled medical image data is applied to a pre-trained contrastive network to determine the one or more image acquisition parameters. A contrastive network is a type of self-supervised neural network that aims to learn representations by contrasting positive pairs (i.e., similar samples) with negative pairs (i.e., dissimilar samples). The contrastive network may be trained based on the positive and negative pairs using a contrastive loss function. The goal is to minimize the loss function, which measures the difference between positive and negative pairs. Contrastive networks may assume that image transformations do not change an image's semantic meaning. Consequently, different augmentations of the same image form a "positive pair," while other images and their augmentations are "negative pairs" relative to the instance. The contrastive network may include a transformer neural network, a convolutional neural network, or a combination thereof. Other types of neural networks are also useful.

In some implementations, the contrastive network includes a simple framework for contrastive learning of visual representations (SimCLR). See, for example, Chen, T., Kornblith, S., Norouzi, M., and Hinton, G. (2020), A simple framework for contrastive learning of visual representations, In International conference on machine learning, pages 1597-1607, which is herein incorporated by reference. SimCLR first learns generic representations of images in an unlabeled dataset, and then it can be fine-tuned with a small number of labeled images to achieve good performance for a given classification task. It outperforms supervised models on the ImageNet benchmark with 100 times fewer labels. However, SimCLR's reliance on very large batch sizes for optimal performance can be computationally demanding.

In other implementations, the contrastive network includes a Simple Siamese Network (SimSiam). See, for example, Chen, X. and He, K. (2021), Exploring simple Siamese representation learning, In Proceedings of the IEEE/CVF conference on computer vision and pattern recognition, pages 15750-15758, which is herein incorporated by reference. SimSiam utilizes Siamese networks without requiring negative samples, enabling it to reduce batch size requirements while learning meaningful representations and preserving model performance.

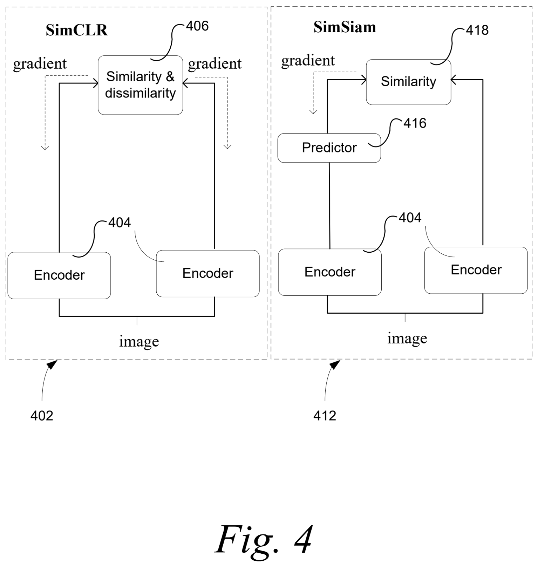

FIG. 4 shows an exemplary SimCLR architecture 402 and an exemplary SimSiam architecture 412. The SimCLR architecture 402 includes first and second branches that process two independently augmented views of the same input image. Each branch includes an encoder network 404 (e.g., residual network or ResNet) which extracts high-dimensional feature representations from the augmented views of the input image. Encoder network 404 may be, for example, a residual neural network (e.g., ResNet-18), which includes convolutional layers with a residual connection. Other types of networks, such as a transformer neural network, a convolutional neural network, or a combination thereof, are also useful. The feature representations are then passed through a small neural network (projection head) to map them to a space where contrastive loss is applied. The contrastive loss function 406 is used to maximize the agreement between the positive pairs (augmented views of the same image) and minimize the agreement between negative pairs (views of different images).

The SimSiam architecture 412 includes first and second branches that process two independently augmented views of the same input image, each branch including an encoder network 404 composed of an encoder (e.g., residual network or ResNet) which extracts high-dimensional feature representations from independently augmented views of the input image. The first branch further includes a prediction projection multilayer perceptron (MLP) 416. The SimSiam architecture 412 maximizes similarity between both sides and does not use negative pairs to optimize performance. The similarity function 418 maximizes the similarity between both branches, while a stop-gradient mechanism applied to the second branch facilitates stability in training and prevents trivial solutions.

SimCLR requires large batch sizes in order to have a sufficiently large pool of negative samples for training, while SimSiam avoids the need for large batch sizes by avoiding negative pairs in the loss function and focusing only on learning the features of positive pairs. Both SimCLR and SimSiam effectively produced MR image sequence classifiers, showing strong performance across various anatomical regions and modalities. These frameworks demonstrate the potential to improve model generalizability while reducing reliance on extensive labeled datasets, which is crucial in medical imaging.

In some implementations, the contrastive network is pre-trained using self-supervised learning based on a training dataset of unlabeled MR images acquired using different types of sequences (e.g., T1, T2, FLAIR, TOF, TraceW, DWI, ADC, GRE, Perfusion). Image data labels are generated automatically, which are further used in subsequent training iterations as ground truths. To maintain the intensity distribution of the training images, the following forms of image data augmentation may be applied to the training dataset: flip, rotation, and elastic deformation. Such custom data augmentation improves the training and ultimate model performance. To reduce computational load during training, each training MR series may be downsized by resampling to a lower resolution (e.g., 84 × 84 pixels). The model is trained to minimize the distance between positive pairs in latent space while maximizing the separation from negative pairs, using various distance metrics within the contrastive loss function. Given computational constraints and the convergence behavior of the training and calibration loss, each pre-training session may be limited to, for example, 50 epochs.

In some implementations, the contrastive network is subsequently fine-tuned in a supervised manner after the self-supervised training. The fine-tuning may be performed using, for example, varying portions (e.g., 0.5% to 100%) of the dataset. Fine-tuning generally refers to further training the contrastive network using a small set of labeled training image data on a particular downstream task (e.g., classifying MR sequence type given labels T1, T2, FLAIR, etc.). The contrastive model may be fine-tuned to regress one or more image acquisition parameters (e.g., TR, TE, flip angle, acceleration). Accordingly, the contrastive network learns general and useful image features during the main self-supervised training, while the subsequent supervised fine-tuning allows the model to use those features to accomplish a particular downstream task.

Returning to FIG. 2, at 206, processing module 106 provides the image acquisition parameters. The image acquisition parameters may be provided via a user interface presented at display device 108. In some implementations, the image acquisition parameters are stored as, for example, annotations in the medical image data, for subsequent retrieval.

Additionally, the medical image data may be processed or presented based on the image acquisition parameters. For example, an image processing software may retrieve the image acquisition parameters, instead of relying on *a priori* knowledge of the type of MR image sequences, to perform denoising or other image processing procedures. As another example, the image processing software may present the same types of image sequences scanned at different times of the same patient together on the same user interface screen. These image sequences may be reviewed together by radiologists to evaluate the progress of diseases over time. As yet another example, the image processing software may present different types of image sequences that are typically reviewed together by radiologists because they provide complementary diagnostic information. For instance, T1-weighted and T2-weighted MR images may be displayed together on the same user interface screen or report as they are frequently used in tandem for diagnosing diseases.

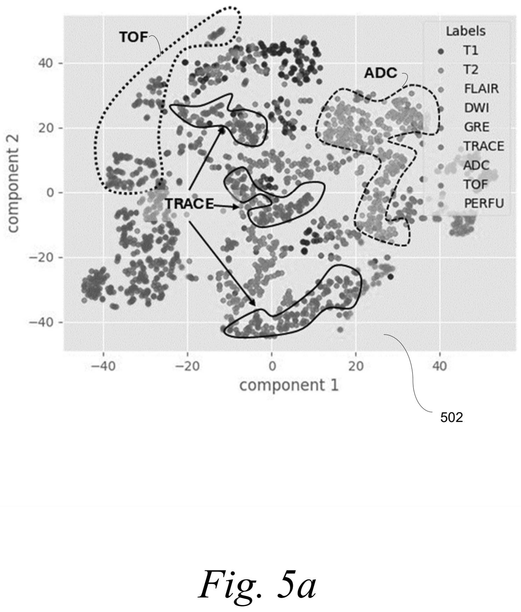

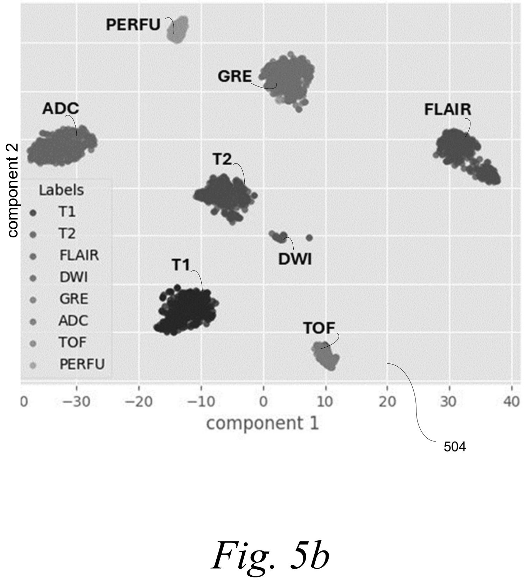

FIGS. 5a-5b show latent space visualization from an exemplary SimCLR model during pre-training to qualitatively measure success of clustering different sequence types together. More particularly, FIG. 5a shows an exemplary visualization 502 of the latent space of the exemplary SimCLR model at epoch 50 of self-supervised pre-training. FIG. 5b shows an exemplary visualization 504 of the latent space of the exemplary SimCLR model after supervised fine-tuning. SimCLR and SimSiam frameworks were implemented using ResNet-18 as the backbone for the pre-training phase with self-supervised pre-training weights. The model's classification performance is fine-tuned in a supervised manner on a brain image dataset for both the SimCLR and SimSiam frameworks. To maintain the intensity distribution of the images, only flip, rotation, and elastic transformations were applied for augmentation. Given computational constraints and the convergence behavior of the training and calibration loss, each pre-training session was limited to 50 epochs. As can be observed in FIG. 5a, a clear clustering of points is observed after 50 epochs, indicating improved feature representation. In FIG. 5b, distinct clusters of points are observed after supervised fine-tuning, indicating the effectiveness of supervised fine-tuning after the self-supervised pre-training.

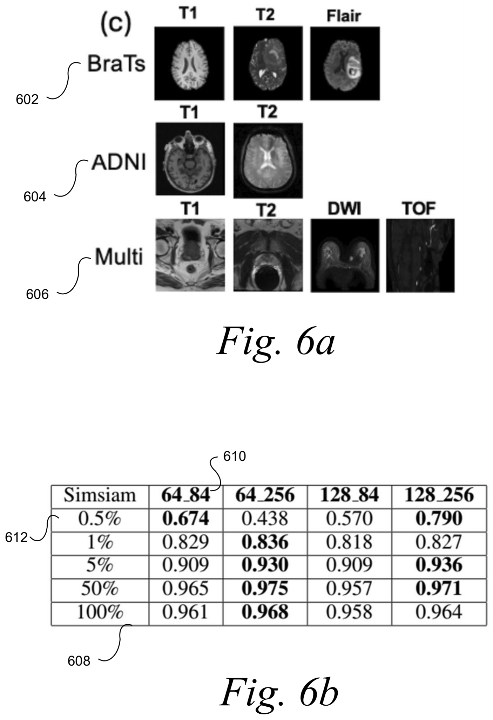

FIG. 6a shows exemplary datasets used for model fine-tuning and evaluation. The exemplary Brain Tumor Segmentation (BraTS) dataset 602 contains T1w, T2w, and FLAIR sequences. The exemplary Alzheimer's Disease Neuroimaging Initiative (ADNI) dataset 604 includes T1w and T2w sequences, while the exemplary multi-organ specific dataset 606 includes T1w and T2w sequences from the Fused Radiology-Pathology Prostate Dataset, DWI sequences from the Breast Cancer Dataset (ACRIN) dataset, and TOF sequences from the Lausanne TOF Aneurysm Cohort. 220 patient images per MR image sequence were extracted from each dataset. To reduce computational load during training, each MR image series was resampled to 80 × 80 pixels. Given the variation in available sequence types across these datasets, the model was retrained for each dataset separately.

FIG. 6b shows classification accuracy results for the new MR image sequences from the extracted datasets. More particularly, table 608 shows the MR sequence classification accuracy of the SimCLR model across varying batch sizes and image resolutions. Each column header 610 represents the format: batch size, resolution. For example, 64_84 indicates a batch size of 64 and an image resolution of 84 × 84. The row header 612 represents the percentage of the dataset that is used for supervised fine-tuning. It can be observed that increasing the resolution to 256 improved classification accuracy compared to a lower resolution of 64.

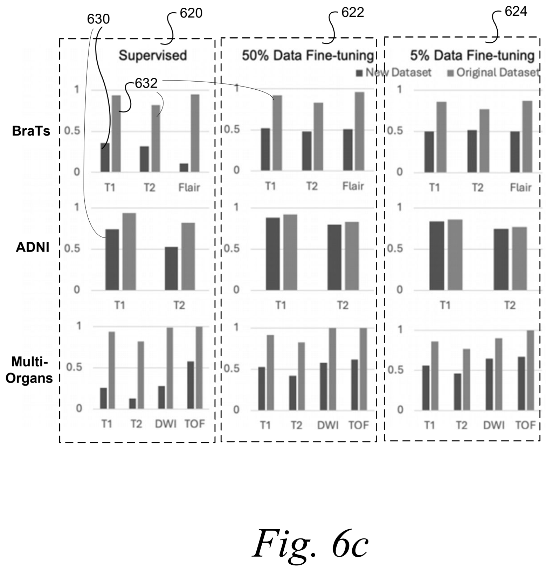

FIG. 6c shows various exemplary classification graphs illustrating the MR sequence classification accuracy performance of an exemplary SimSiam model. Each graph illustrates the classification accuracy performance using an original dataset and a new dataset. The original dataset refers to an internal dataset used for pre-training, supervised training, fine-tuning and testing the SimSiam model. The new dataset refers to each of the external datasets BraTs, ADNI, or multi-organ respectively that is used to test the SimSiam model. First bars 630 depict the classification accuracy on the original dataset test cases, while second bars 632 shows the classification accuracy on the BraTs, ADNI, or multi-organ test datasets respectively to measure the generalizability for the features learned by training on the original internal dataset.

Column 620 of bar graphs depict the classification accuracy of a fully supervised model trained on the original dataset. Column 622 of bar graphs show the performance of a model with unsupervised pre-training followed by supervised fine-tuning using 50% of the original training dataset. Similarly, column 624 of bar graphs show the performance of a model with unsupervised pre-training followed by supervised fine-tuning using 5% of the original training dataset.

As depicted, a noticeable drop in accuracy is observed across three scenarios: (1) fully supervised training, (2) self-supervised pre-training on the original dataset followed by fine-tuning on the original dataset, and (3) self-supervised pre-training on the original dataset followed by fine-tuning on the new datasets. However, the self-supervised method still demonstrates significantly higher classification accuracy than the supervised approach, likely due to the improved generalizability achieved through self-supervised pre-training. It can also be observed that first bars 630 have lower classification accuracies than second bars 632, which can mean that features learned by training only on the original dataset are not fully generalizable to datasets containing new anatomies. However, first bars 630 for the unsupervised and fine-tuning graphs in columns 622 and 624 are higher than the first bars 630 for fully supervised training in column 620, therefore implying that unsupervised feature learning is more generalizable.

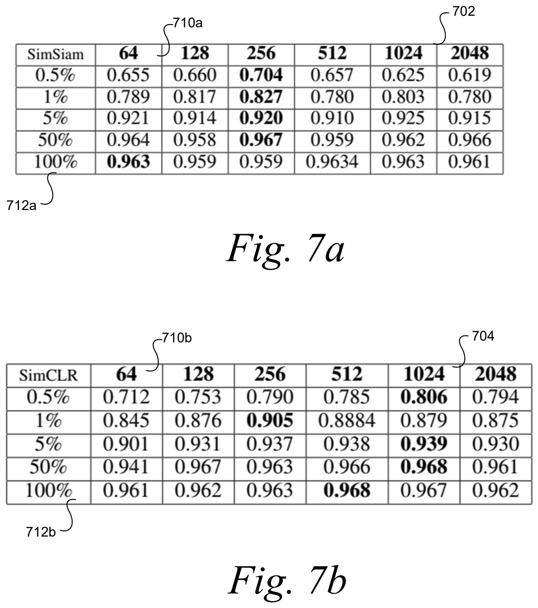

FIG. 7a shows an exemplary table 702 depicting the MR sequence classification performance of an exemplary SimSiam model across varying fine-tuning percentages and batch sizes. FIG. 7b shows an exemplary table 704 depicting the MR sequence classification performance of an exemplary SimCLR model across varying fine-tuning percentages and batch sizes. The column headers 710a-b of the tables (702, 704) represent the batches sizes which range from 64 to 2048. The row headers 712a-b represent the percentage of the dataset that is used for supervised fine-tuning, which range from 0.5% to 100%.

It can be observed that batch size significantly influences the effectiveness of the self-supervised contrastive loss and, subsequently, the pre-training performance. Our results indicate that the optimal batch size is 256 for SimSiam and 1024 for SimCLR across most fine-tuning settings using varying proportions of the dataset. The highest classification accuracy for SimSiam was achieved with 50% of the data and a batch size of 256, while for SimCLR, the highest accuracy was observed with a batch size of 1024 using either 50% or 100% of the dataset for fine-tuning.
Both SimCLR and SimSiam effectively classified MR image sequences, showing strong performance across various anatomical regions and modalities. These frameworks demonstrated the potential to improve model generalizability while reducing reliance on extensive labeled datasets, which is crucial in medical imaging. Various data augmentation and downsizing techniques may be applied to balance performance and computational efficiency.

The following is a list of non-limiting illustrative embodiments disclosed herein:
Illustrative embodiment 1. An image processing system, comprising:
   one or more non-transitory computer-readable media for storing computer-readable program code; and a processor device in communication with the one or more non-transitory computer-readable media, the processor device being operative with the computer-readable program code to perform steps including a) receiving unlabeled medical image data, b) identifying one or more image acquisition parameters by applying the unlabeled medical image data to at least one pre-trained contrastive network, and c) providing the one or more image acquisition parameters.
Illustrative embodiment 2. The image processing system of illustrative embodiment 1 wherein the one or more image acquisition parameters comprise a type of an imaging modality used in acquiring the unlabeled medical image data, one or more parameter settings of the imaging modality, or a combination thereof.
Illustrative embodiment 3. The image processing system of any one of illustrative embodiments 1-2 wherein the one or more image acquisition parameters comprise repetition time (TR), echo time (TE), inversion time, flip angle, acceleration, or a combination thereof.
Illustrative embodiment 4. The image processing system of any one of illustrative embodiments 1-3 wherein the processor device is operative with the computer-readable program code to identify the one or more image acquisition parameters by identifying a magnetic resonance (MR) sequence used in acquiring the unlabeled medical image data.
Illustrative embodiment 5. The image processing system of illustrative embodiment 4 wherein the magnetic resonance sequence comprises T1 weighted, T2 weighted, Fluid-Attenuated Inversion Recovery (FLAIR), Time-of-Flight (TOF), Trace-Weighted Imaging (TraceW), Diffusion-Weighted Imaging (DWI), ADC, Gradient Echo Imaging (GRE), Perfusion MR image sequence, or a combination thereof.
Illustrative embodiment 6. The image processing system of any one of illustrative embodiments 1-5 wherein the at least one pre-trained contrastive network comprises a simple framework for contrastive learning of visual representations (SimCLR).
Illustrative embodiment 7. The image processing system of any one of illustrative embodiments 1-6 wherein the at least one pre-trained contrastive network comprises a Simple Siamese Network (SimSiam).
Illustrative embodiment 8. The image processing system of any one of illustrative embodiments 1-7 wherein the processor device is further operative with the computer-readable program code to train the contrastive network using self-supervised learning based on a training dataset of unlabeled images.
Illustrative embodiment 9. The image processing system of illustrative embodiment 8 wherein the training dataset of unlabeled images are augmented by performing flip, rotation and elastic deformation.
Illustrative embodiment 10. The image processing system of illustrative embodiment 8 wherein the training dataset of unlabeled images are downsized by resampling at a lower resolution.
Illustrative embodiment 11. The image processing system of illustrative embodiment 8 wherein the processor device is further operative with the computer-readable program code to fine-tune the contrastive network in a supervised manner using labeled training image data.
Illustrative embodiment 12. A computer-implemented method for image processing, comprising: a) receiving unlabeled medical image data; b) identifying one or more image acquisition parameters by applying the unlabeled medical image data to at least one pre-trained contrastive network; and c) providing the one or more image acquisition parameters.
Illustrative embodiment 13. The computer-implemented method of illustrative embodiment 12, wherein identifying the one or more image acquisition parameters by applying the unlabeled medical image data to the at least one pre-trained contrastive network comprises classifying the unlabeled medical image data according to a type of magnetic resonance (MR) sequence used to acquire the unlabeled medical image data.
Illustrative embodiment 14. The computer-implemented method of any one of illustrative embodiments 12-13 wherein identifying the one or more image acquisition parameters comprises by applying the unlabeled medical image data to the at least one pre-trained contrastive network comprises applying the unlabeled medical image data to a simple framework for contrastive learning of visual representations (SimCLR).
Illustrative embodiment 15. The computer-implemented method of any one of illustrative embodiments 12-14 wherein identifying the one or more image acquisition parameters comprises by applying the unlabeled medical image data to the at least one pre-trained contrastive network comprises applying the unlabeled medical image data to Simple Siamese Network (SimSiam).
Illustrative embodiment 16. The computer-implemented method of any one of illustrative embodiments 12-15 further comprises training the contrastive network using self-supervised learning based on a training dataset of unlabeled images.
Illustrative embodiment 17. The computer-implemented method of illustrative embodiment 16 further comprises fine-tuning the contrastive network in a supervised manner using labeled training image data.
Illustrative embodiment 18. The computer-implemented method of any one of illustrative embodiments 12-17 wherein providing the one or more image acquisition parameters comprises storing the one or more image acquisition parameters in the medical image data for subsequent retrieval.
Illustrative embodiment 19. The computer-implemented method of any one of illustrative embodiments 12-18 wherein providing the one or more image acquisition parameters comprises processing or presenting the medical image data based on the one or more image acquisition parameters.
Illustrative embodiment 20. One or more non-transitory computer-readable media comprising machine readable instructions, that when executed by one or more processor devices, cause the one or more processor devices to perform method steps comprising: a) receiving unlabeled medical image data; b) identifying one or more image acquisition parameters by applying the unlabeled medical image data to at least one pre-trained contrastive network; and c) providing the one or more image acquisition parameters.

The foregoing examples have been provided merely for the purpose of explanation and are in no way to be construed as limiting of the present framework disclosed herein. While the invention has been described with reference to various embodiments, it is understood that the words, which have been used herein, are words of description and illustration, rather than words of limitation. Further, although the invention has been described herein with reference to particular means, materials, and embodiments, the invention is not intended to be limited to the particulars disclosed herein, rather, the invention extends to all functionally equivalent structures, methods and uses, such as are within the scope of the appended claims. Those skilled in the art, having the benefit of the teachings of this specification, may affect numerous modifications thereto and changes may be made without departing from the scope and spirit of the invention in its aspects.

## Claims

1. An image processing system, comprising:
one or more non-transitory computer-readable media for storing computer-readable program code; and
a processor device in communication with the one or more non-transitory computer-readable media, the processor device being operative with the computer-readable program code to perform steps including
a) receiving unlabeled medical image data,
b) identifying one or more image acquisition parameters by applying the unlabeled medical image data to at least one pre-trained contrastive network, and
c) providing the one or more image acquisition parameters.

2. The image processing system of claim 1 wherein the one or more image acquisition parameters comprise a type of an imaging modality used in acquiring the unlabeled medical image data, one or more parameter settings of the imaging modality, or a combination thereof.

3. The image processing system according to any one of the preceding claims, wherein the one or more image acquisition parameters comprise repetition time (TR), echo time (TE), inversion time, flip angle, acceleration, or a combination thereof.

4. The image processing system according to any one of the preceding claims, wherein the processor device is operative with the computer-readable program code to identify the one or more image acquisition parameters by identifying a magnetic resonance (MR) sequence used in acquiring the unlabeled medical image data.

5. The image processing system of claim 4 wherein the magnetic resonance sequence comprises T1 weighted, T2 weighted, Fluid-Attenuated Inversion Recovery (FLAIR), Time-of-Flight (TOF), Trace-Weighted Imaging (TraceW), Diffusion-Weighted Imaging (DWI), ADC, Gradient Echo Imaging (GRE), Perfusion MR image sequence, or a combination thereof.

6. The image processing system according to any one of the preceding claims, wherein the at least one pre-trained contrastive network comprises a simple framework for contrastive learning of visual representations (SimCLR) and/or a Simple Siamese Network (SimSiam).

7. The image processing system according to any one of the preceding claims, wherein the processor device is further operative with the computer-readable program code to train the contrastive network using self-supervised learning based on a training dataset of unlabeled images.

8. The image processing system of claim 7 wherein the training dataset of unlabeled images are augmented by performing flip, rotation and elastic deformation, and/or wherein the training dataset of unlabeled images are downsized by resampling a resolution.

9. The image processing system according to any one of the preceding claims, wherein the processor device is further operative with the computer-readable program code to fine-tune the contrastive network in a supervised manner using labeled training image data.

10. A computer-implemented method for image processing, comprising:
a) receiving unlabeled medical image data;
b) identifying one or more image acquisition parameters by applying the unlabeled medical image data to at least one pre-trained contrastive network; and
c) providing the one or more image acquisition parameters.

11. The computer-implemented method of claim 10, wherein identifying the one or more image acquisition parameters by applying the unlabeled medical image data to the at least one pre-trained contrastive network comprises classifying the unlabeled medical image data according to a type of magnetic resonance (MR) sequence used to acquire the unlabeled medical image data and/or applying the unlabeled medical image data to a simple framework for contrastive learning of visual representations (SimCLR).

12. The computer-implemented method of claim 11 wherein identifying the one or more image acquisition parameters comprises by applying the unlabeled medical image data to the at least one pre-trained contrastive network comprises applying the unlabeled medical image data to Simple Siamese Network (SimSiam).

13. The computer-implemented according to any one of the preceding method claims, further comprises training the contrastive network using self-supervised learning based on a training dataset of unlabeled images and/or fine-tuning the contrastive network in a supervised manner using labeled training image data.

14. The computer-implemented method according to any one of the preceding method claims, wherein providing the one or more image acquisition parameters comprises storing the one or more image acquisition parameters in the medical image data for subsequent retrieval and/or
wherein providing the one or more image acquisition parameters further comprises processing and/or presenting the medical image data based on the one or more image acquisition parameters.

15. One or more non-transitory computer-readable media comprising machine readable instructions, that when executed by one or more processor devices, cause the one or more processor devices to perform method steps comprising:
a) receiving unlabeled medical image data;
b) identifying one or more image acquisition parameters by applying the unlabeled medical image data to at least one pre-trained contrastive network; and
c) providing the one or more image acquisition parameters.
